# EUROPEAN PATENT SPECIFICATION

(11) **EP 1 638 601 B1**
(45) Date of publication and mention of the grant of the patent: **19.11.2008**
(21) Application number: 04776921.1
(22) Date of filing: 23.06.2004
(51) Int. Cl.: A61K 39/385

(54) **CARRIER PROTEINS FOR VACCINES**
TRÄGERPROTEINE FÜR IMPFSTOFFE
PROTEINES PORTEUSES POUR DES VACCINS

(30) Priority: 23.06.2003 US 480409 P
(43) Date of publication of application: 29.03.2006
(73) Proprietor: Baxter International Inc., Deerfield, IL 60015 (US); Baxter Healthcare S.A., CH-8306 Kanton Zurich (CH)
(72) Inventor: KIM, John, Arbutus, MD 21227 (US); MICHON, Francis, J., Bethesda, MD 20814 (US)
(74) Representative: Ruffles, Graham Keith
(86) International application number: PCT/US2004/020026
(87) International publication number: WO 2005/000346

(56) References cited:
- EP-A- 0 427 347
- WO-A-95/04151
- WO-A-99/15671
- US-A- 5 443 966
- US-A- 5 785 973
- DATABASE BIOSIS [Online] BIOSCIENCES INFORMATION SERVICE, PHILADELPHIA, PA, US; 14 October 2003 (2003-10-14), KIM J ET AL: "Tetanus toxin C-fragment as a universal carrier protein for conjugate vaccines." XP002302424 Database accession no. PREV200400041633 & ABSTRACTS OF THE INTERSCIENCE CONFERENCE ON ANTIMICROBIAL AGENTS AND CHEMOTHERAPY, vol. 43, 2003, page 294, 43RD ANNUAL INTERSCIENCE CONFERENCE ON ANTIMICROBIAL AGENTS AND CHEMOTHERAPY; CHICAGO, IL, USA; SEPTEMBER 14-17, 2003

## Description

The present invention relates to improved carrier proteins for antigen-based vaccines, including polysaccharide-based vaccines.

### BACKGROUND OF THE INVENTION

Conjugation of a polysaccharide to a carrier protein can effectively make that polysaceharide more immunogenic. Tetanus toxoid has been used for decades in this capacity as a carrier, and its safety profile has been established, at least in the context of past uses.

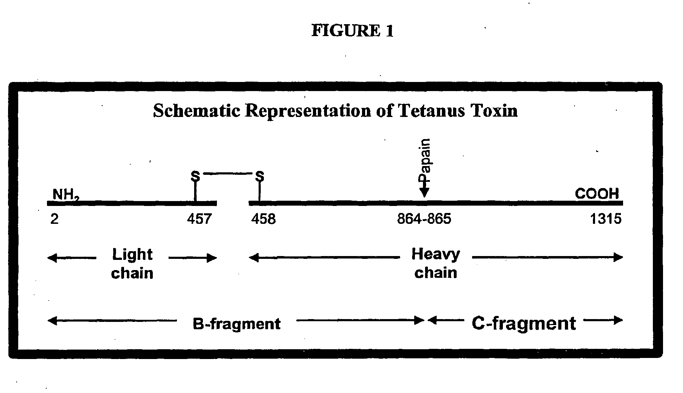

The structural gene for tetanus toxin has been cloned and sequenced. Fairweather et al., J. Bacteriol. 165: 21-27 (1986); Fairweather et al., Nuci. Acid Res. 14: 7809-7812 (1986). These studies have confirmed the structure of tetanus toxin as a 150 kD protein comprising 1315 amino acids. Fragment C, which constitutes the binding portion of native tetanus toxin, is a 52 kD polypeptide generated by papain cleavage of the toxin and corresponds to the 451 amino acids at the C-terminus. See Figure 1.

Tetanus toxoid has been found to contain 2 to 3 universal T-cell epitopes. Demotz et al., J. Immunol 142: 394-402 (1989). This feature makes tetanus toxoid highly effective in humans. Fragment C of the toxoid has been shown to be nontoxic. This fragment also contains at least one of the universal immunogenic T cell epitopes recognized by primed donors. Valmori et al., J. Immunol 149:717-2 1 (1992); Panina-Bordignon et al., Eur. J. Immunol. 19: 2237 (1989).

Capsular polysaccharides (CP) conjugate vaccines targeting a variety of bacterial infections are currently under development and clinical evaluation. The inclusion of multiple CP serotypes combined in a single injection is currently under study. The combination of CP conjugate vaccines into a single multivalent injection, however, can result in competition among the different components and adversely affect the immunogenicity of any individual conjugate. Fattom et al., Vaccine 17:126-33 (1999).

Tetanus toxoid is finding increased use in polysaccharide vaccines. There is now concern arising that the vaccinated population will be over exposed to Tetanus, with the risk of inducting tolerance and/or hypersensitivity throughout the population. For example, injection of mice with an immunogenic dose of carrier followed by immunization with hapten-carrier conjugate selectively suppresses antihapten antibody response. This carrier-induced epitopic suppression may be related to the induction of carrier-specifics T cells which in turn could inhibit selectively antihapten response. Epitopic suppression may induced through the expansion of the clones specific for the carrier epitopes and antigenic competition between hapten and carrier epitopes. Schutze et al., J. Immunol. 37:2635-40 (1989). In humans, it has been demonstrated that prior immunity against a carrier protein modulates the serological response to synthetic conjugate vaccines. Di John et al., Lancet 2 (8677):1415-8 (1989). Barrington et al. (Infect. & Immun. 61: 432-8, 1993) have shown that epitopic suppression of antibody response to *Haemophilus influenzae* type b conjugate vaccine by preimmunization with vaccine components was observed. More recently, Burrage et al. (Infect.& Immun. 70: 4946-54, 2002) have shown some epitopic supression of antibody response to meningococcal C conjugate vaccine by preimmunization with the tetanus carrier protein. In mice, epitopic suppression to the antibody response of pneumococcal and meningococcal polysaceharide-tetanus conjugates was observe after high doses of carrier priming with tetanus toxoid (Peeters et al. Infect & Immun 59:3504-10, 991). Due to these potential adverse consequences, tetanus toxoid should no longer be administered as it has in the past, and therefore improved carriers are needed.

US 5,443,966 describes a tetanus vaccine comprising tetanus toxin Fragment C, in combination with a separate pharmaceutically acceptable carrier and including a further antigen. WO99/15671 discloses a vaccine comprising a tetanus toxin Fragment C fused to a pre-S1 and/or a pre-S2 region of HBV, and a pharmaceutically acceptable carrier. The tetanus toxin and antigen are fused by a hinge linker region. US 5,785,973 describes the use of a immunogenic conjugate comprising a polysaccharide antigen bound to a tetanus toxin epitope, obtained by cleaving the tetanus toxin molecule into fragments. EP 0 427 347 describes a universal peptide carrier corresponding to fragments of the tetanus toxin protein.

None of these documents describe the use of an antigen conjugated to tetanus toxin

Fragment C in the production of a conjugated vaccine, in the obsence of a separate carrier.

### Summary of the invention

According to the present invention, there is provided a method of increasing the immunogenicity of a carbohydrate antigen, the method being set out in claim 1. The invention also provides a conjugated vaccine consisting essentially of a capsular polysaccharide antigen conjugated to tetanus toxin Fragment C. Also provided is the use of such a vaccine in the manufacture of a medicament for immunising a patient against an infection.

Other features of the invention are set forth in the dependent claims.

Also described is a conjugate antigen comprising one or more polysaccharide moieties from at least one target pathogen covalently linked to a tetanus toxoid Fragment C protein moiety. As used herein, "target pathogen" may refer to any exogenous pathogen comprising a polysaccharide epitopes which may be recognized by the immune system of a mammal or avian, such as a bacterial or fungal pathogen.

Also described is a vaccine comprising a conjugate antigen comprising one or more polysaccharide moieties from at least one target pathogen covalently linked to a tetanus toxoid Fragment C protein moiety.

Also described is a method for eliciting an immune response to a target pathogen in a mammal or avian comprising the step of inoculating the mammal or avian with an effective amount of a vaccine comprising a conjugate antigen comprising one or more polysaccharide moieties from at least one target pathogen covalently linked to a tetanus toxoid Fragment C protein moiety. The method may be practice on avians such as chickens, turkeys, emus, ostriches, and other commercially important birds. The method is preferably practiced on mammals such as rodents, equine, bovine, other commercially important herd mammals, canines, felines, other companion animals, and humans. Particularly, the method may be practiced on humans.

Also described is a method for preventing a subsequent infection of a mammal or avian by a target pathogen comprising the step of inoculating the mammal or avian with an effective amount of a vaccine comprising a conjugate antigen comprising one or more polysaccharide moieties from at least one target pathogen covalently linked to a tetanus toxoid Fragment C protein moiety. The method may be praciced on avians such as chickens, turkeys, emus, ostriches, and other commercially important birds. The method is preferably practiced on mammals such as rodents, equines, bovines, other commercially important herd mammals, canines, felines, other companion animals, and humans. Particularly, the method may be practiced on humans.

### Brief Description of the Figures

Figure 1 is a schematic depiction of the Tetanus toxin.

Figure 2 depicts the profile and molecular weight of the native and recombinant TTc.

Figure 3 depicts comparisons of Fragment C, recombinant Fragment C and the Tetanus Toxoid with GCMP conjugates.

Figure 4 depicts comparisons of recombinant Fragment C and the Tetanus Toxoid with meningococcal conjugates.

Figure 5 depicts type-specific IgG elicited by GBSP conjugates with Tetanus Toxoid or recombinant Fragment C.

Figure 6 depicts the efficacy of GBSP conjugates in a neonatal mouse model.

**Detailed Description**

As disclosed for the first time herein, Fragment C of tetanus toxin, often referred to as "TTc", can be used as a carrier protein for polysaccharides, such as for capsular polysaccharide vaccines for protection against bacterial or fungal infections. Fragment C when conjugated to an antigen can increase the immunogenicity of that antigen, meaning that the ability of that antigen to elicit an immune response is enhanced through conjugation to Fragment C. This enhancement often has been referred to as the carrier effect, which in essence transforms the polysaccharide from a T-independent to a T-dependent antigen. The apparent lack of neutralizing Tetanus toxin antibodies in the animals immunized with polysaccharide TTc conjugates and the resulting reduction in recognition of the native tetanus toxoid, together with the apparent conservation of carrier ability, makes TTc an attractive substitute for Tetanus toxoid.

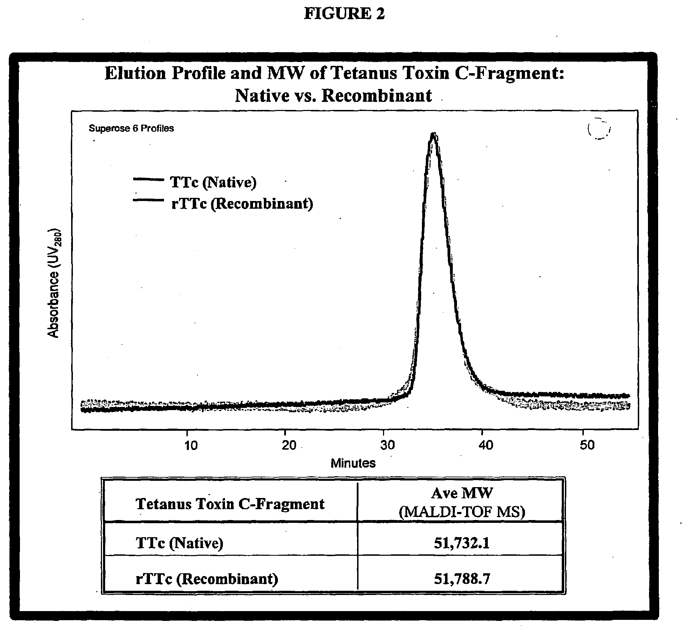

The data presented herein demonstrate that TTc and a recombinant TTc (rTTc) can be used as carrier for polysaccharides and elicit an immune response equivalent to that of Tetanus toxoid in terms of polysaccharide-specific IgG antibodies. See Figure 2. , These antibodies have a high functional activity as measured by the ability to kill the bacteria.

Fragment C (TTc) relates to the carboxyl-terminal portion of about amino acid positions 865-1315, as depicted in Figure 1. Thus, in the context of the present invention, Fragment C refers to separation of the region from at least a portion of the remainder of the whole tetanus toxoid molecule, which can be done by digestion of the toxoid with papain or other proteases or through recombinant expression of the fragment. Recombinant expression of Fragment C is disclosed in U.S. Patent No. 5,443,966. Accordingly, and vaccine comprising an antigen conjugated to Fragment C will lack at least a portion of the non-Fragment C (that is, the B fragment in Figure 1) region.

Of course, various additions, deletions and substitutions can be made to the TTc sequence to yield variants without adversely impacting the carrier capabilities of the molecule. For example, conservative and semi conservative amino acid substitutions can be undertaken. Exemplary conservative amino acid substitutions include, but are not limited to, changes of: alanine to seine; arginine to lysine; asparigine to glutamine or histidine; aspartate to glutamate; cysteine to seine; glutamine to asparigine; glutamate to aspartate; glycine to proline; histidine to asparigine or glutamine; isoleucine to leucine or valine; leucine to valine or isoleucine; lysine to arginine, glutamine, or glutamate; methionine to leucine or isoleucine; phenylalanine to tyrosine, leucine or methionine; seine to threonine; threonine to serine; tryptophan to tyrosine; tyrosine to tryptophan or phenylalanine; valine to isoleucine or leucine. As will be appreciated by those of ordinary skill in the protein engineering arts, the conservative and semi conservative mutations mentioned herein are preferably made to regions outside of the recognized antigenic/immunogenic region of TTc. Suitable regions for mutation for various purposes (e.g., the addition of a polypeptide "tag" for ease of purification of the protein) include the C-terminal and N-terminal regions.

Variants can be created by recombinant techniques employing genomic or cDNA cloning methods. Site-specific and region-directed mutagenesis techniques can be employed. See CURRENT PROTOCOLS IN MOLECULAR BIOLOGY vol. 1, ch. 8 (Ausubel et al. eds., J. Wiley & Sons 1989 & Supp. 1990-93); PROTEIN ENGINEERING (Oxender & Fox eds., A. Liss, Inc. 1987). In addition, linkerscanning and PCR-mediated techniques can be employed for mutagenesis. See PCR TECHNOLOGY (Erlich ed., Stockton Press 1989); CURRENT PROTOCOLS IN MOLECULAR BIOLOGY, vols. 1 & 2, *supra.* Protein sequencing, structure and modeling approaches for use with any of the above techniques are disclosed in PROTEIN ENGINEERING, loc. *cit.* and CURRENT PROTOCOLS IN MOLECULAR BIOLOGY, vols. 1 & 2, *supra.* If desired, other variations to Fragment C can be undertaken by employing combinatorial chemistry, biopanning and/or phage display.

Peptide mimetics of Fragment C can be produced by the approach outlined in Saragovi et al., Science 253: 792-95 (1991) and other articles. Mimetics are peptide-containing molecules which mimic elements of protein secondary structure. See, for example, Johnson et al., "Peptide Turn Mimetics" in BIOTECHNOLOGY AND PHARMACY, Pezzuto et al., Eds., (Chapman and Hall, New York, 1993). The underlying rationale behind the use of peptide mimetics is that the peptide backbone of proteins exists chiefly to orient amino acid side chains in such a way as to facilitate molecular interactions, such as those of antibody and antigen. A peptide mimetic carrier according to the present invention would when administered to a host assist in eliciting an immune response.

Fragment C can be conjugated to the polysaccharide, such as a bacterial or fungal capsular polysaccharide, by methodologies currently in practice. For example, polysaccharides can by deacylated with treatment under slightly basic conditions, and then conjugated to Fragment C via reductive amination with a reducing agent, such a cyanoborohydride anions. See EP 0 658 118 B1. Other conjugation approaches are available to the skilled person, such as those in the examples which follow and those disclosed in W00010599A2, U.S. Published Patent Application No. US 2001/0014332 Al. Another conjugation approach is described by Marburg et al., (J.Am. Chem. Soc., 108, 5282 1986) using bigenic spacers and is also disclosed in US 4,695,624; US 4,830,852; US 4,882,317 and US 5,623,057 which is an improved process over the last three.

Sources of polysaccharide, which will define the type and specificity of vaccine, can be obtained from a wide variety of sources, including bacteria and fungi. Exemplary bacteria include: *Neisseria meningitidis,* including groups A, B, C, Y, W135 and X; *Streptococcus,* including groups A, B, and C; *Pneumococcus,* including types 1,2, 3,4, 6A, 6B, 9, 14, 18F, 19F and 23; *Staphylococcus aureus* including types *5* and 8; *Haemophilus influenzae,* including type b; *Mycobacterium tuberculosis; Campylobacter spp.;* enteroinvasive *Escherichia coli* isolates; *Salmonella typhii; Vibrio cholerae; Shigellaflexneri; Brucella spp.; Francicella tularensis;* and *Yersinia pestis.* Particularly, antigens from *Neisseria meningitidis, Haernophilius influenzae, Pneumococcus, and Streptococcus* may be utilized for conjugation to TTc. Exemplary fungi include among others *Candida albicans, Cryptococcus neoformans,* and *Aspergillus niger.* Using standard carbohydrate chemistries, synthetic. polysaccharides having one or more characteristics of natural CPs can be employed as vaccines.

Fragment C, including variants, can be used as a carrier in much the same way as the present tetanus toxoid is. Pharmaceutically acceptable formulations for the conjugates include aqueous solutions, non-toxic excipients, including salts, preservatives, buffers and the like, as described in UNITED STATES PHARMACOPEIA AND NATIONAL FORMULARY (USP 24-NF 19); REMINGTON'S PHARMACEUTICAL SCIENCES; HANDBOOK ON PHARMACEUTICAL EXCIPIENTS (2d ed., Wade and Weller eds., 1994). Examples of non-aqueous solvents are propylene glycol, polyethylene glycol, vegetable oil and injectable organic esters such as ethyloleate. Aqueous solvents include water, alcoholic/aqueous solutions, saline solutions, parenteral vehicles such as sodium chloride, Ringer's dextrose, etc. Intravenous vehicles include fluid and nutrient replenishers. Preservatives include antimicrobials, antioxidants, chelating agents and inert gases. The pH and exact concentration of the various components of the binding composition are adjusted according to routine skills in the art. *See* GOODMAN AND GILMAN'S THE PHARMACOLOGICAL BASIS FOR THERAPEUTICS (9th ed.). In addition, one or more adjuvants may be added to the vaccine composition. Alum (aluminum hydroxide) is an example of a generally accepted adjuvant for use, although other adjuvants are know to those of skill in the vaccine arts.

Fragment C as part of a polysaccharide conjugate vaccine will usually be administered by a single dose (toddlers and adults) or multiple discrete doses for primary immunization (infant regimen) and as booster shots over a period of time. The specific dose level, and thus the therapeutically-effective amount, for any particular patient can depend on age, weight and sex. Although, as is true with most vaccines, normalized values can be established to develop generalized dosages that are effective across a population of group or subpopulations. Generally, vaccines containing from about 5 to about 100 µg, preferably about 10 to 50 µg, are suitable to elicit effective levels of antibody in young mammals against capsular polysaccharides of pathogenic gram negative or gram positive organisms, and can be further defined via titration and the like. Several small doses given sequentially would be expected to be superior to the same amount of conjugate given as a single injection.

The invention is further described by the following examples, which are illustrative of the invention but do not limit the invention in any manner.

**Example 1: Preparation of rTTc conjugates**

Preparation of Meningococcal C (GCMP), Y (GYMP) and W (GWMP) polysaccharides:

The meningococcal C, Y and W polysaccharides were purified from fermentation broths, containing glucose and yeast extract.

De-O-acetylated (dOA) GCMP was prepared as disclosed in US patent No. 5,425,946.

De-O-acetylated GYMP was prepared as follows:

Polysaccharide capture by UF with a 300 kDa MWCO membrane:

Approximately 13L of cell-free microfiltered fermentation permeate is concentrated by UF to approximately 1 liter using a Biomax 300K Pellicon membrane *(0.5 m²).* The concentrated retentate is diafiltered 12x against 1M NaCl and then 10x against DI water. It is further concentrated to approximately 0.2L and collected.

**Base Hydrolysis of the polysaccharide:**

The 300K retentate solution (ca 5mg PS/mL) was adjusted to a final concentration of 2N NaOH, and placed in an oven set to 80°C for 16-18 hrs. After the reaction mixture had cooled off to less than 50°C, it was diluted into 10L of DI water. After concentration through a 30 kDa MWCO Pellicon membrane, the concentrated retentate was diafiltered 12 times against 1 M NaCl and then 10 times against DI water. It was further concentrated to approximately 0.2L and collected.

**Acid Hydrolysis of the dOA GYMP:**

The retentate solution was transferred to a teflon reaction and sodium acetetate (NaOAc) was added to a final concentration of 0.1 N. The reaction mixture was adjusted to pH5 using 6N HCl and placed in a water bath set to 70°C. It was shaken at 65 rpm until the polysaccharide reached a target MW of approximately 10-20 kDa as measured by SEC-MALLS using a Superose 12 (Pharmacia) column.

**Re-N-Acetylation of the fragmented dOA polysaccharide:**

The pH of the solution was adjusted to 8 with 6N HCI solution, and acetic anhydride was then added dropwise at room temperarure to a final concentration of 0.8 M acetic anhydride. SN NaOH was used to keep the reaction mixture pH between 7 and 9. After completion of the reaction, the pH of the reaction mixture was increased to 13, and the mixture stirred an additional 1.5 hr. The reaction pH was then adjusted to pH 8 with 6N HCI solution. The reaction mixture was poured into 4L of 1 M NaCI, concentrated to approximately 1 L using a Biomax 100K Pellicon membrane (0.5 m²) and the permeate collected. The 100K final permeate is concentrated by UF to approximately 1 liter using a Biomax 5K Pellicon membrane (0.5 m²). The concentrated retentate is diafiltered 10 times against DI water, then concentrated to approximately 0.2L and collected. The fragmented polysaccharide was then activated with sodium metaperiodate to generate aldehyde groups in its sialic acid residues.

The oxidized polysaccharides were then conjugated by seductive amination using sodium cyanoborohydride to tetanus toxoid (Serum Statens Institute, Copenhagen, Denmark) or a recombinant form *(E. coli)* of tetanus toxin C fragment (rTTc) (Roche Molecular Biochemicals, Indianapolis, IN). Biochemical comparison of the recombinant form of tetanus toxin C fragment from Roche and C fragment isolated from tetanus toxin (TTc) after papain digestion (List Biological Laboratories, Inc. Campbell, CA) indicated that both proteins were identical (same AA composition, same MW as measured by MALDI-TOFF, and same elution profile by HPLC). See Figure 2.

Some of the physicochemical characteristics of these conjugates are shown in Table 1 below.

**Table 1**

| Conjugate | [PS] (µg/mL) | [Protein] (µg/mL) | [PS]/[Protein] | Yield PS % | Yield Protein % |
|---|---|---|---|---|---|
| GCMP-rTTc | 347.1 | 441.0 | 0.787 | 27.8 | 88.2 |
| GCMP-TT | 294.2 | 621.2 | 0.474 | 11.3 | 71.4 |
| GYMP-RTTc | 166.4 | 379.0 | 0.439 | 13.3 | 75.8 |
| GYMP-TT | 167 | 656.3 | 0.254 | 25 | 98 |
| GWMP-RTTc | 261.1 | 391.2 | 0.667 | 20.9 | 78.2 |
| GWMP-TT | 69.5 | 200.9 | 0.346 | 8.3 | 60 |

Three of the most clinically important Group B streptococcal (GB S) serotypes (Ia, III and V) polysaccharides were coupled by reductive amination to both tetanus toxoid and rTTc. See U.S. Patent No. 5,993,825. Table 2 sets forth some of their characteristics.

**Table 2**

| Conjugate | [PS] (µg/mL) | [Protein] (µg/mL) | [PS]/[Protein] | Yield PS % | Yield Protein |
|---|---|---|---|---|---|
| GBS Ia-RTTc | 307 | 279 | 1.1 | 25 | 59 |
| GBS Ia-TT | 200 | 240 | 0.8 | 23 | 60 |
| GBS III-RTTc | 273 | 360 | 0.8 | 24 | 72 |
| GBS III-TT | 264 | 460 | 0.6 | 14 | 46 |
| GBS V-RTTc | 341 | 370 | 0.9 | 26 | 84 |
| GBS V-TT | 218 | 305 | 0.7 | 19 | 70 |

Another method of coupling the above polysaccharides to tetanus C fragment, besides reductive amination is described in W00010599A2 patent application. The method involves first re-N-acryloylation of partially or totally de-Nacetylated polysaccharide followed by direct coupling of the activated polysaccharide to the carrier protein at pH 9-10. The chemistry is a Michael addition of the primary amino groups on the protein (u-NH2 of lysinyl residues) to the unsaturated N-acryloyl groups on the polysaccharide. **Example 2: Peclinical Evaluation of rTTc conjugates**

Potency of meningococcal conjugates - Schedule of immunization: 4-6 weeks old Swiss Webster female mice were immunized s.c. at days 0, 28 and 42 with 2 µg of polysaccharide conjugated to either TT (tetanus toxoid) or rTTc. Animals were bled at days 0, 28 and 52. Polysaccharide-specific IgG were measured by ELISA using respective C, Y or W polysaccharides human serum albumin conjugates as the coating antigen and prepared in a similar fashion as for the tetanus conjugate vaccines. Tetanus toxin antibodies raised to the conjugates were measured by ELISA with tetanus toxoid as the coating antigen. Antibody-complement mediated killing of antisera were determined by a Serum bactericidal assay (SBA) using baby rabbit serum as the source of complement.

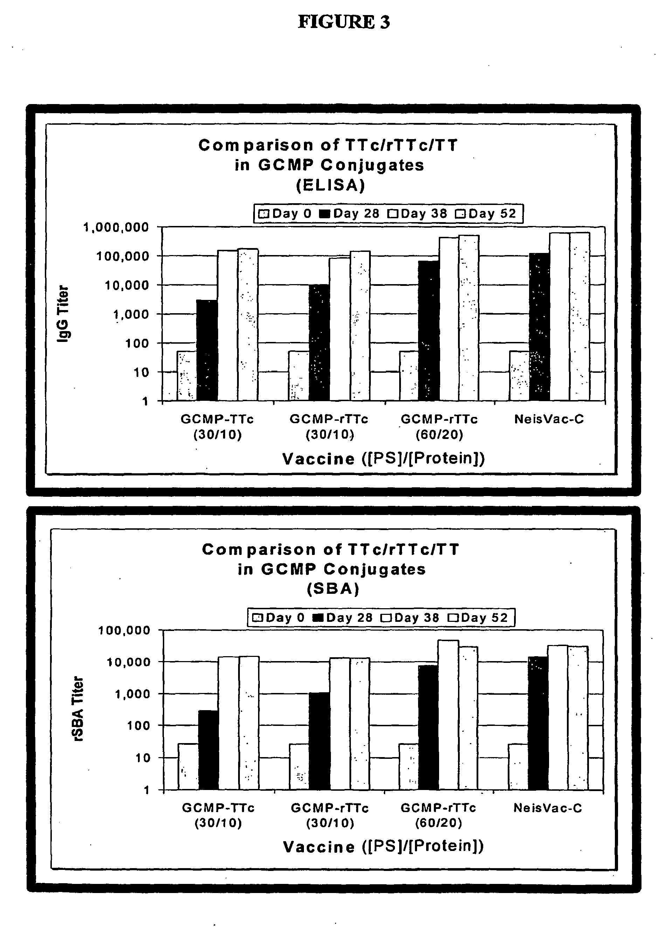

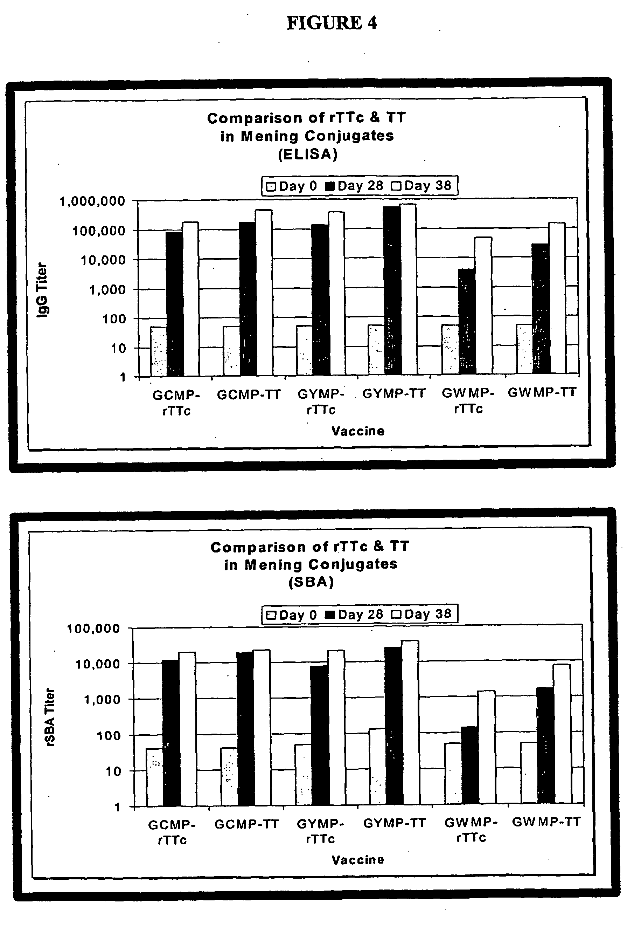

The potency (polysaccharide-specific IgG and serum bactericidal activity) to these conjugates is shown in Figure 3 for a comparison between TTc, rTTc and TT conjugates of GCMP and in Figure 4 for the corresponding immune response to conjugates of GYMP, GWMP and GCMP bound to either TT or rTTc.

As shown in Figure 3 and Figure 4, meningococcal rTTc conjugates do not display significant differences in potency when compared to their corresponding TT constructs. In addition the anti-tetanus response and anti-tetanus C fragment (TTc) was also measured by a series of ELISAs. Significant anti-tetanus response in antisera to GCMP-TT conjugates was basically abolished in sera raised with TTc and rTTc GCMP conjugates as shown in Table 3.

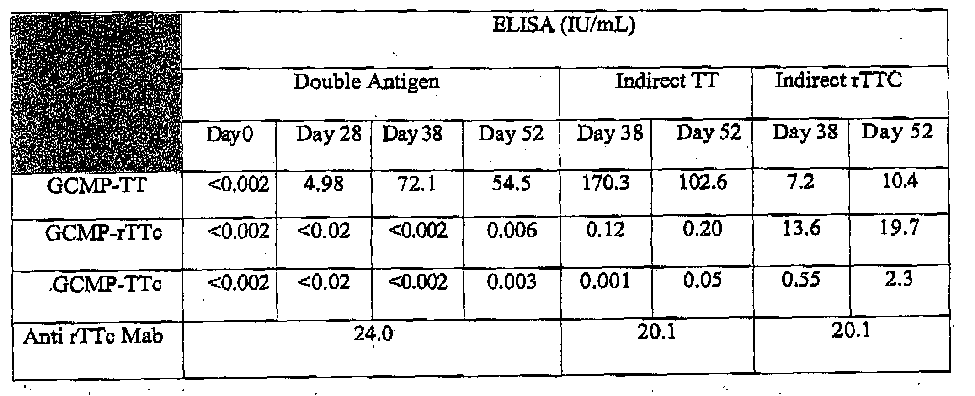

Three assays were used: (a) Double antigen ELISA for Tetanus, (b) Indirect Tetanus IgG ELISA, (Rristiansen et al., APMIS 105:843-53 (1997), (c) Indirect Recombinant Tetanus toxin fragment C IgG ELISA. The assays were incubated at 4 to 8°C over night Assay a and b plates were coated with Tetanus toxoid lot 57 SSI diluted 1:10000 in carbonate buffer pH 9.6, and incubated at 4 to 8°C over night. Plate(s) for assay c was done with reconstituted Recombinant Tetanus toxin Fragment C (rTTc) - lot 85161832, Roche - in 0.1M NaHCO₃ diluted to 1µg/ml in carbonate buffer pH 9.6, and incubated for 2 hours at room temperature. One plate of each coating was applied the same pre-dilutions of samples and standards. Incubation was conducted over night at 4-8°C.

For detection, the Biotin-TT/HRP-Streptavidin system was used for assay a) and HRP-Goat anti Mouse IgG (Fc) 1:5000 was used for assays b) and c). The chromogene O-Phenylene Diamine (OPD) at a concentration of 1mg/mL was used as substrate and the reaction was stopped with 2M H₂50₄. The OD (optical density) was read at 492nm. Data was analyzed using a reference line method.

The anti-rTTc MAb was used to standardize the IgG ELISA data, based on the data from the Double Antigen ELISA. The MAb to rTTc recognizes soluble toxoid as well as coated native Tetanus toxoid and the Fragment C to the same extent. It also indicates that antibodies raised to rTTc can be measured by the three types of ELISAs.

The data presented in Table 3 indicate that a very significant anti-tetanus IgG response was measured in antisera to GCMP-TT conjugates, but this response was practically absent in sera raised against TTc and rTTc GCMP conjugates.

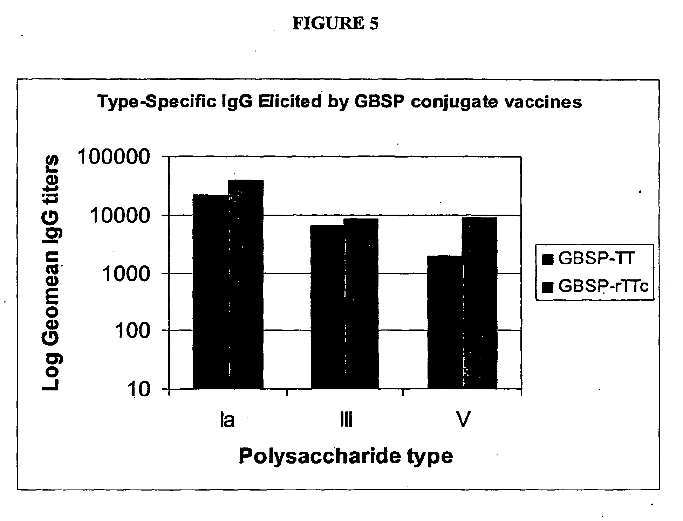

GBS conjugates using either TT or rTTc as the carrier protein were tested for their ability to elicit a protective immune response. The efficacy of the monovalent types Ia, III and V conjugates prepared as described herein was evaluated in the neonatal mice model of Madoff et al. Infec. & Immun. 60:4989-94 (1992). Animals (CD1 female mice) were inoculated or the combination tetravalent vaccine mix, Each animal received 1 p.g of each of the conjugated type-polysaceharide at days 0 and 21. Vaccines were adsorbed on Aluminum hydroxide (Superfos, Denmark). Mice were impregnated at day 21. Neonates were challenged 48 hours following birth with either GBS type Ia (090), GBS type III (M781), or GBS type V (CJ111). The GBS type-specific polysaccharide IgG induced by each individual conjugate are shown in Figure 5. The data indicate that type Ia- and III-rTTc conjugates elicit similar if not better polysaccharide-specific IgG titers than their corresponding TT counterpart, however type V-rTTc conjugates elicit significantly higher type V specific IgG than their corresponding TT.

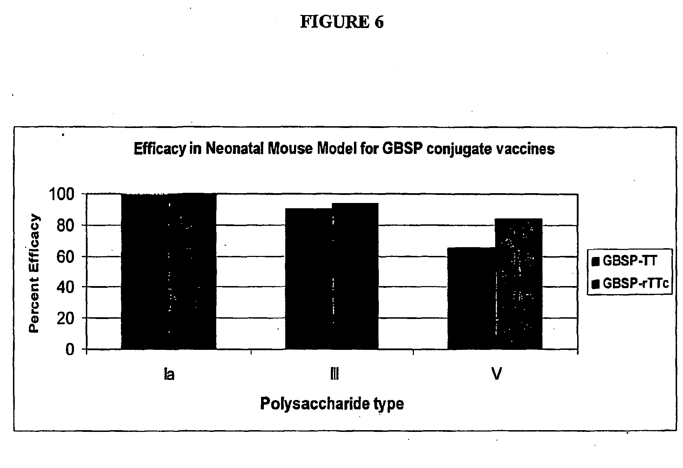

Efficacy results from the neonatal mice challenge correlate well with the antibody surrogate levels, that is, there is a significantly better protection against type V challenge afforded with the V-rTTc conjugates (ca 85% efficacy) than with V-TT (ca 65%), whereas similar protection was provided against challenge with type Ia and III GBS organisms with close to 100% efficacy against Ia, 90-95% against III with either one of the corresponding rTTc or TT conjugates. See Figure 6. It should be noted that in the murine model, type V challenge is the most difficult to overcome following immunization with the polysaccharide conjugate, and thus it is significant that tetanus fragment C as a carrier protein for type V polysaccharide is a demonstrably better carrier protein than the whole tetanus molecule.

The anti-tetanus response generated with the GBS TT and rTTc conjugates as well as the residual TT activity measured in Lf units (measuring flocculation) are shown in Table 4.

**Table 4**

| **Vaccine** | **Mean IU/mi** | **% >=0.01 IU/mI** | **Mean LF/ml** | **% of expected** |
|---|---|---|---|---|
| **PBS** | **Nd** | **nd** | **<0.007** | **NA** |
| **GBSP_{Ia}-TT** | **40.0** | **100** | **0.261** | ***6.5*** |
| **GBSP_{Ia}rTTc** | **0.0034** | **10** | **<0.007** | **NA** |
| **GBSP_{III}-TT** | **19.0** | **100** | **0.094** | **2.4** |
| **GBSP_{III}rTTc** | **<0.002** | **0** | **<0.007** | **NA** |
| **GBSPᵥTT** | **27.8** | **100** | **0.059** | **1.5** |
| **GBSPᵥ-rTTc** | **0.0124** | **30** | **<0.007** | **NA** |

This study is based on the assumption that the protein concentration is 10 µg/mL, the expected concentration is set to 4 Lf/mL for TT and 10Lf/mL for rTTc.

Data from Table 4 indicate that the anti-tetanus IgG response in the sera induced by the rTTc conjugates is dramatically reduced when compared to the response induced by the corresponding TT conjugates. In addition, the number of tetanus toxin B-cell epitopes retained after conjugation of the C fragment to the GBS polysaccharides is virtually reduced to zero when compared to the same B-cell epitopes retained in the corresponding TT conjugates. Accordingly, the data presented in Table 3 and Table 4 support and demonstrate further advantages of the present invention in overcoming potential carrier-induced epitopic suppression of the antibody response to polysaccharide conjugate vaccine containing tetanus as the carrier protein.

## Claims

1. A method of increasing the immunogenicity of a carbohydrate antigen, comprising:
conjugating the antigen to tetanus toxin Fragment C to yield a conjugated vaccine, wherein upon administration of the conjugated vaccine to a patient the Fragment C increases the potency of the antigen, with essentially no increase in the protective potency to tetanus toxin.

2. The method according to claim 1, wherein the antigen is a capsular polysaccharide from a bacterium.

3. The method according to claim 2, wherein the bacterium is selected from the group consisting of Meningococcus group A, B, C, Y, W135 and X; Streptococcus group A, B, and C; Pneumococcus types, 1, 2, 3, 4, 6A, 6B, 9, 14, 18F, 19F and 23; Staphylococcus aureus types 5 and 8 and Haemophilus influenzae type b.

4. The method according to claim 1, wherein the antigen is a capsular polysaccharide from a fungus.

5. The method according to claim 4, wherein the fungus is selected from the group consisting of Candida albicans and Cryptococcus neoformans.

6. Use of a conjugated vaccine consisting essentially of antigen conjugated to tetanus toxin Fragment C, where said antigen is a capsular polysaccharide, in the manufacture of a medicament for immunizing a patient against an infection.

7. The use according to claim 6, wherein the antigen is a capsular polysaccharide from a bacterium.

8. The use according to claim 7, wherein the bacterium is selected from the group consisting of Meningococcus groups A, B, C, Y, W135 and X; Streptococcus groups A, B, and C; Pneumococcus types, 1,2,3,4, 6A, 6B, 9, 14, 18F, 19F and 23; Staphylococcus aureus types 5 and 8 and Haemophilus influenzae type b.

9. The use according to claim 6, wherein the antigen is a capsular polysaccharide from a fungus.

10. The use according to claim 9, wherein the fungus is selected from the group consisting of Candida albicans and Cryptococcus neoformans.

11. A conjugated vaccine consisting essentially of antigen conjugated to tetanus toxin Fragment C, wherein said antigen is a capsular polysaccharide.

12. The conjugated vaccine according to claim 11, wherein the antigen is a capsular polysaccharide from a bacterium.

13. The conjugated vaccine according to claim 12, wherein the bacterium is selected from the group consisting of Meningococcus group A, B, C, Y, W135 and X; Streptococcus group A, B, and C; Pneumococcus types 1, 2, 3, 4, 6A, 6B, 9, 14, 18F, 19F and 23; Staphylococcus aureus types 5 and 8 and Haemophilus influenzae type b.

14. The conjugated vaccine according to claim 11, wherein the antigen is a capsular polysaccharide from a fungus.

15. The conjugated vaccine according to claim 14, wherein the fungus is selected from the group consisting of Candida albicans and Cryptococcus neoformans.

## Patentansprüche

1. Verfahren zum Erhöhen der Immunisierungsstärke eines Kohlenhydrat-Antigens, umfassend:
Konjugieren des Antigens mit dem Tetanustoxin-Fragment C, um einen konjugierten Impfstoff zu ergeben, wobei bei Verabreichung des konjugierten Impfstoffes an einen Patienten das Fragment C die Wirksamkeit des Antigens mit im wesentlichen keiner Erhöhung in der Schutzwirksamkeit gegen das Tetanustoxin erhöht.

2. Verfahren nach Anspruch 1, wobei das Antigen ein Kapselpolysaccharid von einem Bakterium ist.

3. Verfahren nach Anspruch 2, wobei das Bakterium aus der Gruppe, bestehend aus der Meningococcus-Gruppe A, B, C, Y, W135 und X; der Streptococcus-Gruppe A, B und C; den Pneumococous-Typen 1, 2, 3, 4, 6A, 6B, 9, 14, 18F, 19F und 23; den Staphylococcus-aureus-Typen 5 und 8 und dem Haemophilus-influenzae-Typ b, ausgewählt ist.

4. Verfahren nach Anspruch 1, wobei das Antigen ein Kapselpolysaccharid von einem Pilz ist.

5. Verfahren nach Anspruch 4, wobei der Pilz aus der Gruppe, bestehend aus Candida albicans und Cryptococcus neoformans, ausgewählt ist.

6. Verwendung eines konjugierten Impfstoffes, bestehend im wesentlichen aus Antigen, konjugiert mit dem Tetanustoxin-Fragment C, wobei das Antigen ein Kapselpolysaccharid ist, bei der Herstellung eines Medikaments zum Immunisieren eines Patienten gegen eine Infektion.

7. Verwendung nach Anspruch 6, wobei das Antigen ein Kapselpolysaccharid von einem Bakterium ist.

8. Verwendung nach Anspruch 7, wobei das Bakterium aus der Gruppe, bestehend aus der Meningococcus-Gruppe A, B, C, Y, W135 und X; der Streptococcus-Gruppe A, B und C; den Pneumococcus-Typen 1, 2, 3, 4, 6A, 6B, 9, 14, 18F, 19F und 23; den Staphylococcus-aureus-Typen 5 und 8 und dem Haemophilus-influenazae-Typ b, ausgewählt ist.

9. Verwendung nach Anspruch 6, wobei das Antigen ein Kapselpolysaccharid von einem Pilz ist.

10. Verwendung nach Anspruch 9, wobei der Pilz aus der Gruppe, bestehend aus Candida albicans und Cryptococcus neoformans, ausgewählt ist.

11. Konjugierter Impfstoff, bestehend im wesentlichen aus Antigen, konjugiert mit dem Tetanustoxin-Fragment C, wobei das Antigen ein Kapselpolysaccharid ist.

12. Konjugierter Impfstoff nach Anspruch 11, wobei das Antigen ein Kapselpalysaccharid von einem Bakterium ist.

13. Konjugierter Impfstoff nach Anspruch 12, wobei das Bakterium aus der Gruppe, bestehend aus der Meningococcus-Gruppe A, B, C, Y, W135 und X; der Streptococcus Gruppe A, B und C; den Pneumococcus-Typen 1, 2, 3, 4, 6A, 6B, 9, 14, 18F, 19F und 23; den Staphylococcus-aureus-Typen 5 und 8 und dem Haemophilus-influenzae-Typ b, ausgewählt ist.

14. Konjugierter Impfstoff nach Anspruch 11, wobei das Antigen ein Kapselpolysaccharid von einem Pilz ist.

15. Konjugierter Impfstoff nach Anspruch 14, wobei der Pilz aus der Gruppe, bestehend aus Candida albicans und Cryptococcus neoformans, ausgewählt ist.

## Revendications

1. Procédé destiné à augmenter l'immunogénicité d'un antigène d'hydrate de carbone, comprenant:
conjuguer l'antigène au fragment C de la toxine du tétanos pour donner un vaccin conjugué, où lors de l'administration du vaccin conjugué à un patient, le fragment C augmente le pouvoir de l'antigène essentiellement sans augmenter le pouvoir protecteur envers la toxine du tétanos.

2. Le procédé selon la revendication 1, dans lequel l'antigène est un polysaccharide capsulaire provenant d'une bactérie.

3. Le procédé selon la revendication 2, dans lequel la bactérie est sélectionnée parmi le groupe consistant en les groupes A, B, C, Y, W135 et X de *Meningococcus;* en les groupes A, B et C de *Streptococcus;* en les types 1, 2, 3, 4, 6A, 6B, 9, 14, 18F, 19F et 23 de *Pneumococcus;* en les types 5 et 8 de *Staphylococcus aureus* et en le type b *d'Haemophilus influenzae_{.}*

4. Le procédé selon la revendication 1, dans lequel l'antigène est un polysaccharide capsulaire provenant d'un champignon.

5. Le procédé selon la revendication 4, dans lequel le champignon est sélectionné parmi le groupe consistant en *Candida albicans* et *Cryptococcus neoformans.*

6. Utilisation d'un vaccin conjugué consistant essentiellement en un antigène conjugué au fragment C de la toxine du tétanos, où ledit antigène est un polysaccharide capsulaire, dans la fabrication d'un médicament destiné à immuniser un patient contre une infection.

7. L'utilisation selon la revendication 6, où l'antigène est un polysaccharide capsulaire provenant d'une bactérie.

8. L'utilisation selon la revendication 7, où la bactérie est sélectionnée parmi le groupe consistant en les groupes A, B, C, Y, W135 et X de *Meningococcus;* en les groupes A, B et C de *Streptococcus;* en les types 1, 2, 3, 4, 6A, 6B, 9, 14, 18F, 19F et 23 de *Pneumococcus*; en les types 5 et 8 de *Staphylococcus aureus* et en le type b *d'Haemophilus influenzae.*

9. L'utilisation selon la revendication 6, où l'antigène est un polysaccharide capsulaire provenant d'un champignon.

10. L'utilisation selon la revendication 9, où le champignon est sélectionné parmi le groupe consistant en *Candida albicans* et *Cryptococcus neoformans,*

11. Vaccin conjugué consistant essentiellement en un antigène conjugué au fragment C de la toxine du tétanos, où ledit antigène est un polysaccharide capsulaire.

12. Le vaccin conjugué selon la revendication 11, dans lequel l'antigène est un polysaccharide capsulaire provenant d'une bactérie.

13. Le vaccin conjugué selon la revendication 12, dans lequel la bactérie est sélectionnée parmi le groupe consistant en les groupes A, B, C, Y, W135 et X de *Meningococcus*; en les groupes A, B et C de *Streptococcus;* en les types 1, 2, 3, 4, 6A, 6B, 9, 14, 18F, 19F et 23 de *Pneumococcus;* en les types 5 et 8 de *Staphylococcus aureus* et en le type b *d'Haemophilus influenzae.*

14. Le vaccin conjugué selon la revendication 11, dans lequel l'antigène est un polysaccharide capsulaire provenant d'un champignon.

15. Le vaccin conjugué selon la revendication 14, dans lequel le champignon est sélectionné parmi le groupe consistant en *Candida albicans* et *Cryptococcus neoformans.*
